# EUROPEAN PATENT APPLICATION

(11) **EP 3 904 422 A1**
(43) Date of publication of application: **03.11.2021**
(21) Application number: 19903722.7
(22) Date of filing: 17.12.2019
(51) Int. Cl.: C08G 65/333, A61Q 1/02, A61Q 1/04, A61Q 1/12, A61Q 1/14, A61Q 15/00, A61Q 17/04, A61Q 19/10, C08K 5/01, C08K 5/101, C08L 75/08, C08L 83/04, C08L 91/00, A61Q 5/00, A61Q 5/02, A61Q 5/04, A61Q 5/06, A61Q 5/08, A61Q 5/12, C08G 18/48, A61K 8/37

(54) **URETHANE POLYMER AND OIL COMPOSITION CONTAINING SAME**

(30) Priority: 25.12.2018 JP 2018240931
(71) Applicant: ADEKA CORPORATION, Arakawa-ku Tokyo 116-8554 (JP)
(72) Inventor: MATSUKURA, Noriyoshi, Tokyo 116-8554 (JP); MIYAZONO, Keitarou, Tokyo 116-8554 (JP); MASUKAWA, Ryo, Tokyo 116-8554 (JP)
(74) Representative: Kador & Partner PartG mbB
(86) International application number: PCT/JP2019/049346
(87) International publication number: WO 2020/137679

(57) **Abstract**

The present invention provides: a urethane polymer having a structure represented by general formula (1) below; an oil composition in which the urethane polymer is dissolved; and a cosmetic composition containing the oil composition. 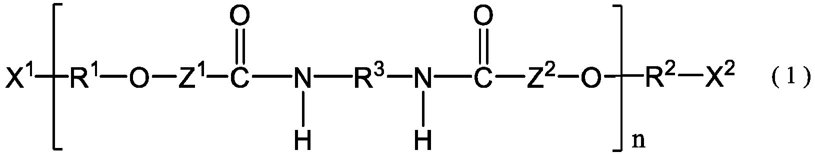 (In the formula, R¹ and R² each independently denote an alkylene group having 5 to 40 carbon atoms, Z¹ and Z² each denote an oxyalkylene group represented by general formula (2) or (3) disclosed in the description, R³ denotes a diisocyanate residue, X¹ denotes a hydrogen atom or a group represented by general formula (4) disclosed in the description, X² denotes a hydrogen atom or a group represented by general formula (5) disclosed in the description, and n denotes a number from 1 to 20, provided that n is 1 if X¹ and X² are each a hydrogen atom.)

## Description

### Technical Field

The present invention relates to a urethane polymer that is able to be used in a variety of fields, such as coating materials, adhesives, fuels, lubricants, foods and cosmetic products; an oil composition containing same; and a cosmetic composition containing the oil composition.

### Background Art

Viscosity modifiers, thickening agents and modifiers are used in a variety of products, such as coating materials, adhesives, fuels, lubricants, foods and cosmetic products, in order to adjust physical properties, such as feel before and after use, and viscosity. Natural viscosity modifiers, such as carboxymethyl cellulose and hydroxyethyl cellulose; alkali thickening type viscosity modifiers in which thickening is accomplished by an alkali, such as poly(acrylic acid) and poly(acrylic acid)-containing copolymers; and urethane type viscosity modifiers, such as urethane-modified polyethers, are known as examples of viscosity modifiers. Of these, many types of urethane type viscosity modifier are produced and used for reasons such as being able to impart a variety of characteristics to products to which the viscosity modifiers are added, and exhibiting excellent long-term stability and durability.

Patent Document 1 discloses a urethane type viscosity modifier which is hardly affected by temperature, can retain viscosity within a certain range under a variety of conditions, is hardly affected by changes in external temperature, and exhibits excellent workability. Patent Document 2 discloses a urethane type polymer which exhibits characteristics similar to those of existing urethane type viscosity modifiers, exhibits long term storage stability similar to that of alkali thickening type viscosity modifiers, and can be used in a variety of fields, such as coating materials, adhesives, foods and cosmetic products.

However, the viscosity modifier disclosed in Patent Document 1 and the urethane type polymer disclosed in Patent Document 2 exhibit a function when dissolved in an aqueous solution, but do not dissolve in oily components, hence are not able to adjust physical properties of oily components, such as feel before and after use, and viscosity. In addition, Patent Document 3 proposes a urethane type oil thickener which dissolves in oils such as greases, mineral oils and synthetic oils, and which can form a gel-like oil, a jelly-like oil or a gummi-like gel.

However, the urethane type oil thickener of Patent Document 3 cannot produce a thickened product or a gel when used with a silicone oil, a derivative thereof, or the like, and is therefore restricted in terms of being unable to achieve satisfactory characteristics in cases when the urethane type oil thickener is used in a variety of products such as coating materials, adhesives, fuels, lubricants, foods and cosmetic products, which contain a variety of components as raw materials, and there remains a need for a urethane polymer that can be widely used in a variety of fields and products.

### Citation List

### Patent Document

[Patent Document 1] Japanese Patent Application Publication No. H09-71767
[Patent Document 2] Republication of International Patent Publication No. WO 2014/084174
[Patent Document 3] Japanese Patent Application Publication No. 2011-102256

### Summary of Invention

### Technical Problem

The problem to be solved by the present invention is to provide: a urethane polymer which is soluble in a variety of oily components, such as hydrocarbon oils, silicone oils and ester oils, and can therefore modify or improve characteristics, such as viscosity and feel before and after use, of a variety of products that contain oily components, such as coating materials, adhesives, fuels, lubricants, foods and cosmetic products; an oil composition in which the urethane polymer is dissolved; and a cosmetic composition containing the oil composition.

### Solution to Problem

As a result of diligent research, the inventors of the present invention discovered a urethane polymer which is soluble in a variety of oily components and can therefore modify or improve characteristics, such as viscosity and feel before and after use, of oily components and a variety of products that contain oily components, and thereby completed the present invention. That is, the present invention is a urethane polymer having a structure represented by general formula (1) below. 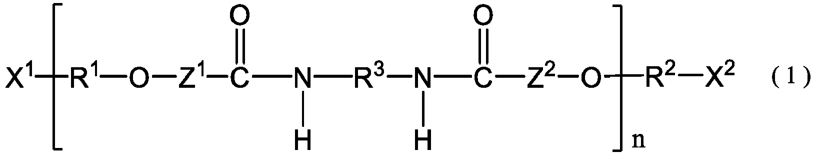 (in the formula, R¹ and R² each independently denote an alkylene group having 5 to 40 carbon atoms, Z¹ and Z² each denote an oxyalkylene group represented by general formula (2) or (3) below, R³ denotes a diisocyanate residue, X¹ denotes a hydrogen atom or a group represented by general formula (4) below, X² denotes a hydrogen atom or a group represented by general formula (5) below, and n denotes a number from 1 to 20, provided that n is 1 if X¹ and X² are each a hydrogen atom.) 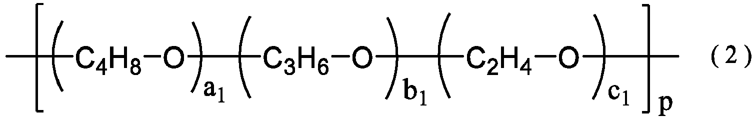 (in the formula, a₁, b₁ and c₁ denote a ratio a₁:b₁:c₁ = 1-0.6:0-0.4:0-0.4 (a sum of a₁, b₁ and c₁ is 1), and p denotes a number from 4 to 60. Moreover, arrangement of structural units may be block-like or random.) 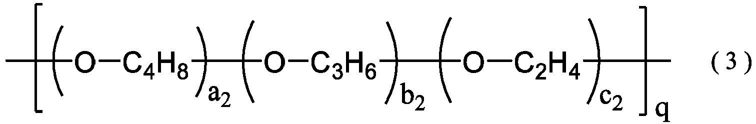 (in the formula, a₂, b₂ and c₂ denote a ratio a₂:b₂:c₂ = 1-0.6:0-0.4:0-0.4 (a sum of a₂, b₂ and c₂ is 1), and q denotes a number from 4 to 60. Moreover, arrangement of structural units may be block-like or random.) 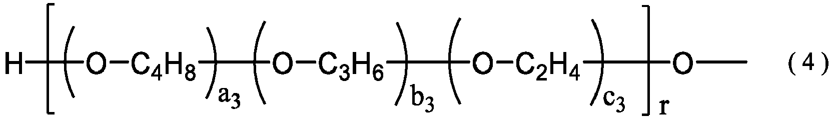 (in the formula, a₃, b₃ and c₃ denote a ratio a₃:b₃:c₃ = 1-0.6:0-0.4:0-0.4 (a sum of a₃, b₃ and c₃ is 1), and r denotes a number from 4 to 60. Moreover, arrangement of structural units may be block-like or random.) 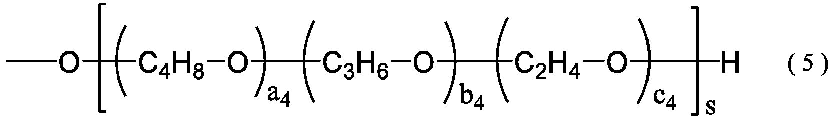 (in the formula, a₄, b₄ and c₄ denote a ratio a₄:b₄:c₄ = 1-0.6:0-0.4:0-0.4 (ae sum of a₄, b₄ and c₄ is 1), and s denotes a number from 4 to 60. Moreover, arrangement of structural units may be block-like or random.)

### Advantageous Effects of Invention

The urethane polymer of the present invention is soluble in a variety of oily components including hydrocarbon oils, silicone oils and ester oils, and can therefore modify or improve characteristics, such as viscosity and feel before and after use, of oily components and a variety of products that contain oily components. The present invention also provides: an oil composition, the viscosity and feel before and after use of which are modified or improved by using the urethane polymer of the present invention; and a variety of products, such as coating materials, adhesives, fuels, lubricants, foods and cosmetic products, the viscosity and feel before and after use of which are modified or improved by using the urethane polymer of the present invention.

### Description of Embodiments

The urethane polymer of the present invention has a structure represented by general formula (1) below. 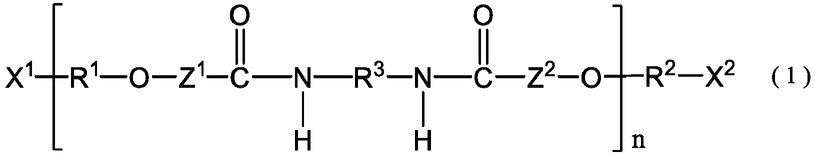 (in the formula, R¹ and R² each independently denote an alkylene group having 5 to 40 carbon atoms, Z¹ and Z² each denote an oxyalkylene group represented by general formula (2) or (3) below, R³ denotes a diisocyanate residue, X¹ denotes a hydrogen atom or a group represented by general formula (4) below, X² denotes a hydrogen atom or a group represented by general formula (5) below, and n denotes a number from 1 to 20, provided that n is 1 if X¹ and X² are each a hydrogen atom.)

In general formula (1), R¹ and R² each independently denote an alkylene group having 5 to 40 carbon atoms. Examples of this type of alkylene group include straight chain alkylene groups having 5 to 40 carbon atoms, such as pentylene groups, hexylene groups, heptylene groups, octylene groups, nonylene groups, decylene groups, undecylene groups, dodecylene groups, tridecylene groups, tetradecylene groups, pentadecylene groups, hexadecylene groups, heptadecylene groups, octadecylene groups, nonadecylene groups, eicosylene groups, heneicosylene groups, docosylene groups, tricosylene groups, tetracosylene groups, pentacosylene groups, hexacosylene groups, heptacosylene groups, octacosylene groups, nonacosylene groups, triacontylene groups, hentriacontylene groups, dotriacontylene groups, tritriacontylene groups, tetratriacontylene groups, pentatriacontylene groups, hexatriacontylene groups, heptatriacontylene groups, octatriacontylene groups, nonatriacontylene groups and tetracontylene groups; and branched alkylene groups having 5 to 40 carbon atoms, such as isopentylene groups, isohexylene groups, isoheptylene groups, isooctylene groups, isononylene groups, isodecylene groups, isoundecylene groups, isododecylene groups, isotridecylene groups, isotetradecylene groups, isopentadecylene groups, isohexadecylene groups, isoheptadecylene groups, isooctadecylene groups, isononadecylene groups, isoeicosylene groups, isoheneicosylene groups, isodocosylene groups, isotricosylene groups, isotetracosylene groups, isopentacosylene groups, isohexacosylene groups, isoheptacosylene groups, isooctacosylene groups, isononacosylene groups, isotriacontylene groups, isohentriacontylene groups, isodotriacontylene groups, isotritriacontylene groups, isotetratriacontylene groups, isopentatriacontylene groups, isohexatriacontylene groups, isoheptatriacontylene groups, isooctatriacontylene groups, isononatriacontylene groups and isotetracontylene groups. Of these alkylene groups, straight chain and branched alkylene groups having 6 to 36 carbon atoms are preferred from the perspective of solubility of the urethane polymer in a variety of oily components. More specifically, it is preferable for R¹ and R² to be hexylene groups, dodecylene groups, octadecylene groups or alkylene groups having 36 carbon atoms.

R³ in general formula (1) denotes a residue of a diisocyanate compound, and more specifically denotes a residue after a diisocyanate compound mentioned below has reacted (a structure obtained by removing 2 isocyanate groups of a diisocyanate compound). From the perspective of solubility in a variety of oily components, R³ is preferably a hydrocarbon group which has 2 to 20 carbon atoms and which may have a functional group, more preferably an aliphatic hydrocarbon group having 4 to 16 carbon atoms or an alicyclic hydrocarbon group having 4 to 16 carbon atoms, and further preferably an alicyclic hydrocarbon group having 6 to 12 carbon atoms. More specifically, R³ is preferably a residue of an aliphatic diisocyanate having a hydrocarbon group with 2 to 20 carbon atoms or a residue of an alicyclic diisocyanate having a hydrocarbon group with 4 to 20 carbon atoms from the perspective of solubility in a variety of oily components. More specifically, R³ is more preferably a residue of hexamethylene diisocyanate, dicyclohexylmethane-4,4'-diisocyanate or isophorone diisocyanate. In addition, from the perspective of being able to maintain satisfactory solubility even in cases where the mass ratio of the urethane polymer of the present invention is high relative to a variety of oily components, R³ is more preferably a residue of dicyclohexylmethane-4,4'-diisocyanate or isophorone diisocyanate, and further preferably a residue of isophorone diisocyanate.

In general formula (1), n denotes a number from 1 to 20. However, n is 1 if X¹ and X² are each a hydrogen atom. Moreover, n being a number from 1 to 20 means a single compound in which the value of n falls within this range or a mixture of compounds in which the value of n falls within this range. The present invention preferably contains a compound for which the value of n is 1 from the perspective of effectively achieving the advantageous effect of the present invention. From the perspective of solubility of the urethane polymer of the present invention in a variety of oily components, the value of n is preferably 1 to 10. In addition, from the perspective of being able to maintain satisfactory solubility even in cases where the mass ratio of the urethane polymer of the present invention is high relative to a variety of oily components, it is more preferable for the value of n to be only 1.

In general formula (1), Z¹ and Z² are oxyalkylene groups represented by general formulae (2) and (3) respectively. 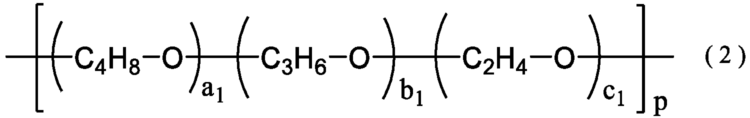 (in the formula, a₁, b₁ and c₁ express the ratio a₁:b₁:c₁ = 1-0.6:0-0.4:0-0.4 (the sum of a₁, b₁ and c₁ is 1), and p denotes a number from 4 to 60. Moreover, the arrangement of structural units may be block-like or random.) 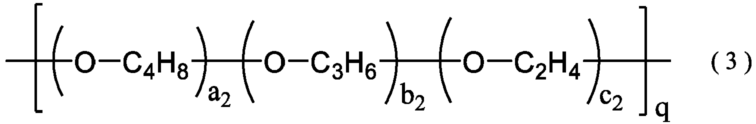 (in the formula, a₂, b₂ and c₂ express the ratio a₂:b₂:c₂ = 1-0.6:0-0.4:0-0.4 (the sum of a₂, b₂ and c₂ is 1), and q denotes a number from 4 to 60. Moreover, the arrangement of structural units may be block-like or random.)

In general formula (2), a₁, b₁ and c₁ express the ratio a₁:b₁:c₁ = 1-0.6:0-0.4:0-0.4 (the sum of a₁, b₁ and c₁ is 1). Moreover, the arrangement of structural units may be block-like or random. From the perspective of solubility of the urethane polymer of the present invention in a variety of oily components, it is preferable for a₁:b₁:c₁ to be 1-0.6:0-0.4:0, more preferable for a₁:b₁:c₁ to be 1-0.8:0-0.2:0, and further preferable for a₁:b₁:c₁ to be 1:0:0 (that is, for Z¹ to be an oxyalkylene group consisting only of an oxybutylene unit).

In general formula (2), p denotes a number from 4 to 60. From the perspective of solubility of the urethane polymer of the present invention in a variety of oily components, p is preferably a number from 6 to 40, and more preferably a number from 8 to 30.

In general formula (3), a₂, b₂ and c₂ express the ratio a₂:b₂:c₂ = 1-0.6:0-0.4:0-0.4 (the sum of a₂, b₂ and c₂ is 1). Moreover, the arrangement of structural units may be block-like or random. From the perspective of solubility of the urethane polymer of the present invention in a variety of oily components, it is preferable for a₂:b₂:c₂ to be 1-0.6:0-0.4:0, more preferable for a₂:b₂:c₂ to be 1-0.8:0-0.2:0, and further preferable for a₂:b₂:c₂ to be 1:0:0 (that is, for Z² to be an oxyalkylene group consisting only of an oxybutylene unit).

In general formula (3), q denotes a number from 4 to 60. From the perspective of solubility of the urethane polymer of the present invention in a variety of oily components, q is preferably a number from a 6 to 40, and more preferably a number from 8 to 30.

From the perspective of solubility in a variety of oily components, the urethane polymer of the present invention is preferably such that in general formula (1), Z¹ is a group in which a₁:b₁:c₁ = 1:0:0 in general formula (2) (that is, Z¹ is an oxyalkylene group consisting only of an oxybutylene unit) and Z² is a group in which a₂:b₂:c₂ = 1:0:0 in general formula (3) (that is, Z² is an oxyalkylene group consisting only of an oxybutylene unit).

In general formula (1), X¹ denotes a hydrogen atom or a group represented by general formula (4) below. 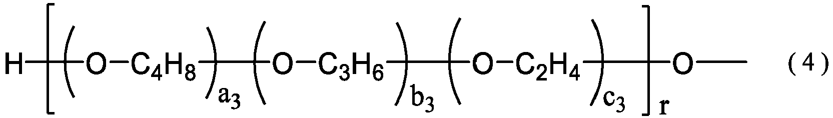 (in the formula, a₃, b₃ and c₃ express the ratio a₃:b₃:c₃ = 1-0.6:0-0.4:0-0.4 (the sum of a₃, b₃ and c₃ is 1), and r denotes a number from 4 to 60. Moreover, the arrangement of structural units may be block-like or random.)

In general formula (4), a₃, b₃ and c₃ express the ratio a₃:b₃:c₃ = 1-0.6:0-0.4:0-0.4 (the sum of a₃, b₃ and c₃ is 1). Moreover, the arrangement of structural units may be block-like or random. From the perspective of solubility of the urethane polymer of the present invention in a variety of oily components, it is preferable for a₃:b₃:c₃ to be 1-0.6:0-0.4:0, more preferable for a₃:b₃:c₃ to be 1-0.8:0-0.2:0, and further preferable for a₃:b₃:c₃ to be 1:0:0 (that is, for X¹ to be an oxyalkylene group consisting only of an oxybutylene unit).

In general formula (4), r denotes a number from 4 to 60. From the perspective of solubility in a variety of oily components, r is preferably a number from 6 to 40, more preferably a number from 8 to 30, and further preferably a number from 8 to 16.

In general formula (1), X² denotes a hydrogen atom or a group represented by general formula (5) below. 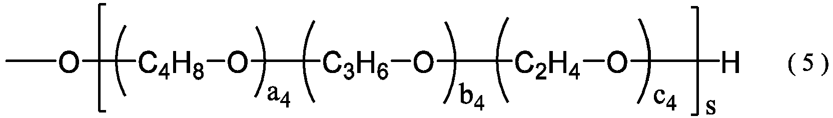 (in the formula, a₄, b₄ and c₄ express the ratio a₄:b₄:c₄ = 1-0.6:0-0.4:0-0.4 (the sum of a₄, b₄ and c₄ is 1), and s denotes a number from 4 to 60. Moreover, the arrangement of structural units may be block-like or random.)

In general formula (5), a₄, b₄ and c₄ express the ratio a₄:b₄:c₄ = 1-0.6:0-0.4:0-0.4 (the sum of a₄, b₄ and c₄ is 1). Moreover, the arrangement of structural units may be block-like or random. From the perspective of solubility of the urethane polymer of the present invention in a variety of oily components, it is preferable for a₄:b₄:c₄ to be 1-0.6:0-0.4:0, more preferable for a₄:b₄:c₄ to be 1-0.8:0-0.2:0, and further preferable for a₄:b₄:c₄ to be 1:0:0 (that is, for X² to be an oxyalkylene group consisting only of an oxybutylene unit).

In general formula (5), s denotes a number from 4 to 60. From the perspective of solubility in a variety of oily components, s is preferably a number from 6 to 40, more preferably a number from 8 to 30, and further preferably a number from 8 to 16.

From the perspective of solubility in a variety of oily components, the urethane polymer of the present invention is preferably such that in general formula (1), X¹ is a hydrogen atom or a group in which a₃:b₃:c₃ = 1:0:0 in general formula (4) (that is, X¹ is an oxyalkylene terminal group consisting only of an oxybutylene unit) and X² is a group in which a₄:b₄:c₄ = 1:0:0 in general formula (5) (that is, X² is an oxyalkylene terminal group consisting only of an oxybutylene unit).

Among urethane polymers having a structure represented by general formula (1) in the present invention, a urethane polymer in which X¹ and X² are each a hydrogen atom and n is 1 in general formula (1) is preferred from the perspective of solubility in a variety of oily components.

The weight average molecular weight of the urethane polymer of the present invention is not particularly limited, but from the perspective of solubility of the urethane polymer of the present invention in a variety of oily components is, for example, preferably 600 to 200,000, more preferably 800 to 50,000, further preferably 1000 to 20,000, and further preferably 1500 to 12,000. Moreover, all weight average molecular weights in the present invention can be measured by means of gel permeation chromatography (GPC) and determined in terms of styrene.

The urethane polymer of the present invention is soluble in a variety of oily components, such as hydrocarbon oils, silicone oils, ester oils and higher alcohols.
Moreover, in the present invention, the statement "the urethane polymer of the present invention is soluble in an oily component" means that when the urethane polymer and an oily component are mixed, no white turbidness or sedimentation is visually observed in the mixture, and the urethane polymer of the present invention is completely dissolved (the mixture is transparent).

Hydrocarbon oils able to dissolve the urethane polymer of the present invention are not particularly limited, but examples thereof include liquid paraffin, squalane, pristane, ozokerite, paraffins, ceresin, Vaseline, polyisobutene, polyisoprene, isodecane, isododecane, isohexadecane, n-pentane, isopentane, n-hexane, isohexane, kerosene, decalin, tetralin and microcrystalline waxes.

Silicone oils able to dissolve the urethane polymer of the present invention are not particularly limited, but examples thereof include chain-like silicone oils such as dimethylpolysiloxane, diphenylpolysiloxane, diphenylsiloxyphenyltrimethicone and octamethyltrisiloxane; cyclic silicone oils such as decamethylcyclotetrasiloxane, dodecamethylcyclotetrasiloxane, octamethylcyclotetrasiloxane, cyclopentasiloxane, dodecamethylcyclopentasiloxane, octamethylcyclopentasiloxane, decamethylcyclohexasiloxane, dodecamethylcyclohexasiloxane and octamethylcyclohexasiloxane; and modified silicone oils such as alkyl-modified dimethylpolysiloxanes, polyether-modified dimethylpolysiloxanes, fatty acid-modified polysiloxanes, higher alcohol-modified polysiloxanes, amino-modified polysiloxanes and fluorine-modified polysiloxanes.

Ester oils able to dissolve the urethane polymer of the present invention are not particularly limited, but examples thereof include synthetic ester oils such as ethyl acetate, butyl acetate, hexyl acetate, decyl acetate, butyl propionate, cetyl octanoate, hexyldecyl dimethyloctanoate, isononyl isononanoate, isotridecyl isononanoate, ethyl laurate, hexyl laurate, myristyl myristate, isopropyl myristate, isopropyl palmitate, 2-ethylhexyl palmitate, 2-hexyldecyl palmitate, 2-heptylundecyl palmitate, decyl oleate, oleyl oleate, octyldodecyl oleate, isocetyl stearate, glycerol stearate, butyl stearate, ethylhexyl hydroxystearate, ethylene glycol stearate, octyl oxystearate, diethyl phthalate, triethyl citrate, 2-ethylhexyl succinate, diisopropyl sebacate, di-2-ethylhexyl sebacate, cetyl lactate, myristyl lactate, diisobutyl adipate, 2-hexyldecyl adipate, di-2-heptylundecyl adipate, cetyl ethyl hexanoate, triethylhexanoin, polyglyceryl-2 triisostearate, neopentyl glycol dioctanoate, neopentyl glycol dicaprate and pentaerythrityl tetraisostearate; and plant-based and animal-based ester oils such as lanolin, mink oil, cocoa butter, coconut oil, palm kernel oil, camellia oil, sesame oil, castor oil and olive oil.

Higher alcohols able to dissolve the urethane polymer of the present invention are not particularly limited, but examples thereof include cetyl alcohol, isostearyl alcohol, lauryl alcohol, hexadecyl alcohol and octadecyl alcohol.

Because the urethane polymer of the present invention is soluble in a variety of oily components, properties of oily components, such as viscosity and feel before and after use, can be adjusted.

The method for producing the urethane polymer of the present invention is not particularly limited, but it is possible to, for example, react raw materials including a monohydric alcohol compound having an alkyl group with 5 to 40 carbon atoms and/or a dihydric alcohol compound having an alkylene group with 5 to 40 carbon atoms, an alkylene oxide having an alkylene group with 2 to 4 carbon atoms, which must include an alkylene oxide having an alkylene group with 4 carbon atoms, and a diisocyanate compound using a well-known method.

Examples of monohydric alcohol compounds having an alkyl group with 5 to 40 carbon atoms include monohydric alcohols having an alkyl group with 5 to 40 carbon atoms. In addition, examples of dihydric alcohol compounds having an alkylene group with 5 to 40 carbon atoms include dihydric alcohols having an alkylene group with 5 to 40 carbon atoms. It is possible to use a single monohydric alcohol compound or dihydric alcohol compound or two or more types thereof, and is possible to use a combination of a monohydric alcohol compound and a dihydric alcohol compound. From the perspective of solubility of an obtained urethane polymer in a variety of oily components, it is preferable to use a monohydric alcohol having an alkyl group with 6 to 36 carbon atoms, a dihydric alcohol having an alkylene group with 6 to 36 carbon atoms, or a mixture of these. In addition, from the perspective of being able to maintain satisfactory solubility even in cases where the mass ratio of the urethane polymer of the present invention is high relative to a variety of oily components, it is preferable to use at least one type of monohydric alcohol having an alkyl group with 6 to 36 carbon atoms, and more preferable to use at least one type of monohydric alcohol having an alkyl group with 10 to 20 carbon atoms. Moreover, by using only a monohydric alcohol compound as the alcohol compound, a urethane polymer in which X¹ and X² are each a hydrogen atom and n is 1 in general formula (1) is obtained.

In general, examples of alkylene oxides having an alkylene group with 2 to 4 carbon atoms include an alkylene oxide having an alkylene group with 2 carbon atoms (ethylene oxide), an alkylene oxide having an alkylene group with 3 carbon atoms (propylene oxide) and an alkylene oxide having an alkylene group with 4 carbon atoms (butylene oxide). However, cases where an alkylene oxide is used as a raw material in the production of the urethane polymer of the present invention are characterized by using an alkylene oxide having an alkylene group with 2 to 4 carbon atoms, which must include alkylene oxide having an alkylene group with 4 carbon atoms. From the perspective of solubility of an obtained urethane polymer in a variety of oily components, the molar ratio of an alkylene group having 4 carbon atoms, an alkylene group having 3 carbon atoms and an alkylene group having 2 carbon atoms is preferably 1-0.6:0-0.4:0-0.4, more preferably 1-0.6:0-0.4:0, further preferably 1-0.8:0-0.2:0, and further preferably 1:0:0 (in all of these cases, the sum of the values in the ratio is 1) .

Examples of diisocyanate compounds include aliphatic diisocyanates such as tetramethylene diisocyanate, pentamethylene diisocyanate, hexamethylene diisocyanate, 2,2-dimethylpentane diisocyanate, 3-methoxyhexane diisocyanate, octaamethylene diisocyanate, 2,2,4-trimethylpentane diisocyanate, nonamethylene diisocyanate, decamethylene diisocyanate, 3-butoxyhexane diisocyanate and dodecamethylene diisocyanate; alicyclic diisocyanates such as cyclohexane diisocyanate, methylcyclohexane diisocyanate, ethylcyclohexane diisocyanate, propylcyclohexane diisocyanate, dicyclohexylmethane-2,2'-diisocyanate, dicyclohexylmethane-2,4'-diisocyanate, dicyclohexylmethane-4,4'-diisocyanate and isophorone diisocyanate; and aromatic diisocyanates such as meta-phenylene diisocyanate, para-phenylene diisocyanate, 2,4-tolylene diisocyanate, 2,6-tolylene diisocyanate, dimethylbenzene diisocyanate, ethylbenzene diisocyanate, isopropylbenzene diisocyanate, meta-xylylene diisocyanate, para-xylylene diisocyanate, 1,4-naphthalene diisocyanate, 1,5-naphthalene diisocyanate, 2,6-naphthalene diisocyanate and 2,7-naphthalene diisocyanate. Among these diisocyanate compounds, from the perspective of solubility of an obtained urethane polymer in a variety of oily components, it is preferable to use an aliphatic diisocyanate, an alicyclic diisocyanate or a mixture thereof, and more specifically, it is more preferable to use hexamethylene diisocyanate, dicyclohexylmethane-4,4'-diisocyanate, isophorone diisocyanate or a mixture thereof, further preferable to use dicyclohexylmethane-4,4'-diisocyanate, isophorone diisocyanate or a mixture thereof, and further preferable to use isophorone diisocyanate.

A urethane polymer having a structure represented by general formula (1) can be produced by, for example, reacting raw materials including a monohydric alcohol component having an alkyl group with 5 to 40 carbon atoms and/or a dihydric alcohol compound having an alkylene group with 5 to 40 carbon atoms, an alkylene oxide having an alkylene group with 2 to 4 carbon atoms, which must include an alkylene oxide having an alkylene group with 4 carbon atoms, and a diisocyanate compound. Specifically, the urethane polymer is produced by a method comprising forming a urethane bond through a reaction between a hydroxyl group in the monohydric alcohol compound having an alkyl group with 5 to 40 carbon atoms and/or dihydric alcohol compound having an alkylene group with 5 to 40 carbon atoms and an epoxy group in the alkylene oxide having an alkylene group with 2 to 4 carbon atoms, which must include an alkylene oxide having an alkylene group with 4 carbon atoms, and a reaction between a hydroxyl group derived from the reaction product of the alkylene oxide having an alkylene group with 2 to 4 carbon atoms, which must include an alkylene oxide having an alkylene group with 4 carbon atoms, and an isocyanate group in the diisocyanate compound.

In the production method mentioned above, a method for reacting a monohydric alcohol compound having an alkyl group with 5 to 40 carbon atoms and/or a dihydric alcohol compound having an alkylene group with 5 to 40 carbon atoms, an alkylene oxide having an alkylene group with 2 to 4 carbon atoms, which must include an alkylene oxide having an alkylene group with 4 carbon atoms, and a diisocyanate compound is not particularly limited. For example, a urethane polymer may be produced by mixing a monohydric alcohol compound having an alkyl group with 5 to 40 carbon atoms and/or a dihydric alcohol compound having an alkylene group with 5 to 40 carbon atoms, an alkylene oxide having an alkylene group with 2 to 4 carbon atoms, which must include an alkylene oxide having an alkylene group with 4 carbon atoms, and a diisocyanate compound all together and carrying out a reaction, or by reacting a monohydric alcohol compound having an alkyl group with 5 to 40 carbon atoms and/or a dihydric alcohol compound having an alkylene group with 5 to 40 carbon atoms with an alkylene oxide having an alkylene group with 2 to 4 carbon atoms, which must include an alkylene oxide having an alkylene group with 4 carbon atoms, so as to obtain an alkylene oxide-added alcohol compound, and then reacting the alkylene oxide-added alcohol compound with a diisocyanate compound. In addition, it is possible to subject an alkylene oxide having an alkylene group with 2 to 4 carbon atoms, which must include an alkylene oxide having an alkylene group with 4 carbon atoms, to a polymerization reaction with a water molecule or the like so as to obtain an alkylene glycol polymer (a polyalkylene glycol), and then mix a monohydric alcohol compound having an alkyl group with 5 to 40 carbon atoms and/or a dihydric alcohol compound having an alkylene group with 5 to 40 carbon atoms and a diisocyanate compound all together and carry out a reaction or react with a monohydric alcohol compound having an alkyl group with 5 to 40 carbon atoms and/or a dihydric alcohol compound having an alkylene group with 5 to 40 carbon atoms so as to obtain an alkylene oxide-added alcohol compound and then react with a diisocyanate compound. Among these production methods, from the perspective of solubility of an obtained urethane polymer in a variety of oily components, it is preferable to produce a urethane polymer by reacting a monohydric alcohol compound having an alkyl group with 5 to 40 carbon atoms and/or a dihydric alcohol compound having an alkylene group with 5 to 40 carbon atoms with an alkylene oxide having an alkylene group with 2 to 4 carbon atoms, which must include an alkylene oxide having an alkylene group with 4 carbon atoms, so as to obtain an alkylene oxide-added alcohol compound, and then react the an alkylene oxide-added alcohol compound with a diisocyanate compound.

Moreover, in any of these production methods, in cases where an alkylene oxide having an alkylene group with 4 carbon atoms and an alkylene oxide having an alkylene group with 2 or 3 carbon atoms are used as alkylene oxides having an alkylene group with 2 to 4 carbon atoms, it is possible to subject all of these simultaneously to a reaction or a polymerization reaction in isolation with a monohydric alcohol compound having an alkyl group with 5 to 40 carbon atoms and/or a dihydric alcohol compound having an alkylene group with 5 to 40 carbon atoms, but it is also possible to subject the alkylene oxide having an alkylene group with 4 carbon atoms to a reaction or a polymerization reaction in isolation with a monohydric alcohol compound having an alkyl group with 5 to 40 carbon atoms and/or a dihydric alcohol compound having an alkylene group with 5 to 40 carbon atoms and then subject the alkylene oxide having an alkylene group with 2 or 3 carbon atoms to a reaction or a polymerization reaction.

The reactions mentioned above are not particularly limited as long as conditions are such that the raw materials react, and it is possible to carry out the reactions either by introducing the entire amount of raw materials all at once or by dividing the raw materials into several portions and introducing the portions separately. For example, a specific example is a method comprising introducing raw materials of the components into a reaction system, either all at once or as several separate portions, mixing at a temperature of 40 to 160°C, and preferably 80 to 160°C, and a pressure of 0.1 to 20 kg/cm², and preferably 1.0 to 10 kg/cm², and maintaining this environment for a period of 30 minutes to 10 hours until the reaction is complete.

In the reactions mentioned above, the blending ratio of the raw materials is not particularly limited, but in a production method in which, for example, a monohydric alcohol compound having an alkyl group with 5 to 40 carbon atoms and/or a dihydric alcohol compound having an alkylene group with 5 to 40 carbon atoms, an alkylene oxide having an alkylene group with 2 to 4 carbon atoms, which must include an alkylene oxide having an alkylene group with 4 carbon atoms, and a diisocyanate compound are introduced all at once and reacted, the molar ratio of hydroxyl groups in the monohydric alcohol compound having an alkyl group with 5 to 40 carbon atoms and/or dihydric alcohol compound having an alkylene group with 5 to 40 carbon atoms, epoxy groups in the alkylene oxide having an alkylene group with 2 to 4 carbon atoms, which must include an alkylene oxide having an alkylene group with 4 carbon atoms, and isocyanate groups in the diisocyanate compound is preferably 1:4-60:0.10-2.0, more preferably 1:6-40:0.15-1.0, and further preferably 1:8-30:0.20-0.9, from the perspective of solubility of the obtained urethane polymer in a variety of oily components.

In addition, in cases where a urethane polymer is produced by reacting a monohydric alcohol compound having an alkyl group with 5 to 40 carbon atoms and/or a dihydric alcohol compound having an alkylene group with 5 to 40 carbon atoms with an alkylene oxide having an alkylene group with 2 to 4 carbon atoms, which must include an alkylene oxide having an alkylene group with 4 carbon atoms, so as to obtain an alkylene oxide-added alcohol and then reacting the alkylene oxide-added alcohol with a diisocyanate compound, the molar ratio of hydroxyl groups in the monohydric alcohol compound having an alkyl group with 5 to 40 carbon atoms and/or dihydric alcohol compound having an alkylene group with 5 to 40 carbon atoms and epoxy groups in the alkylene oxide having an alkylene group with 2 to 4 carbon atoms, which must include an alkylene oxide having an alkylene group with 4 carbon atoms, is preferably 1:4-60, more preferably 1:6-40, and further preferably 1:8-30, and the ratio of hydroxyl groups in the obtained alkylene oxide-added alcohol and isocyanate groups in the diisocyanate compound is preferably 1:0.1-2.0, more preferably 1:0.15-1.0, and further preferably 1:0.2-0.9, from the perspective of solubility of the obtained urethane polymer in a variety of oily components. By setting the molar ratio of hydroxyl groups in the alkylene oxide-added alcohol and isocyanate groups in the diisocyanate compound to be preferably 1:0.1-2.0, more preferably 1:0.15-1.0, and further preferably 1:0.2-0.9, it is easy to control the structure of the obtained urethane polymer and, more specifically, it is easy to set the value of n in general formula (1) to fall within a specific range and it is possible to obtain a urethane polymer of the present invention that exhibits uniform solubility in a variety of oily components.

In addition, it is possible to use a catalyst in the reactions mentioned above in order to facilitate the reactions. Examples of catalysts include strong acids such as sulfuric acid and toluene sulfonic acid; metal halides such as titanium tetrachloride, hafnium chloride, zirconium chloride, aluminum chloride, gallium chloride, indium chloride, iron chloride, tin chloride and boron fluoride; alkali metal and alkaline metal hydroxides, alcoholates and carbonates, such as sodium hydroxide, potassium hydroxide, lithium methylate and sodium carbonate; metal oxides such as aluminum oxide, calcium oxide, barium oxide and sodium oxide; organometallic compounds such as tetraisopropyl titanate, dibutyl tin dichloride, dibutyl tin oxide; and dibutyl tin bis(2-ethylhexylthioglycolate); and soaps such as sodium acetate, potassium acetate, sodium propionate, potassium propionate, sodium octylate, potassium octylate, sodium laurate and potassium laurate. The blending quantity of these catalysts is not particularly limited, but is approximately 0.01 to 1 mass% relative to the total mass of raw materials. Moreover, although the reactions progress without use of a catalyst, use of a catalyst increases the reaction rate, and therefore achieves the advantageous effect of shortening the reaction time.

In the reactions mentioned above, other raw materials may be used as long as characteristics of the obtained urethane polymer (solubility in oily components) are not impaired, but from the perspective of solubility of an obtained urethane polymer in a variety of oily components, it is preferable to produce a urethane polymer by reacting only three types of raw material, namely a monohydric alcohol compound having an alkyl group with 5 to 40 carbon atoms and/or a dihydric alcohol compound having an alkylene group with 5 to 40 carbon atoms, an alkylene oxide having an alkylene group with 2 to 4 carbon atoms, which must include an alkylene oxide having an alkylene group with 4 carbon atoms, and a diisocyanate compound.

The oil composition of the present invention comprises the urethane polymer of the present invention and an oily component that includes at least one type selected from the group consisting of a hydrocarbon oil, a silicone oil and an ester oil. For example, the hydrocarbon oils, silicone oils and ester oils mentioned above can be used as this hydrocarbon oil, silicone oil or ester oil. According to the present invention, it is possible to obtain an oil composition whose viscosity and feel before and after use are modified or improved. In one embodiment of this oil composition, the oily component includes at least one type of hydrocarbon oil. In another embodiment, the oily component includes at least one type of silicone oil. In another embodiment, the oily component includes at least one type of ester oil.

In the oil composition of the present invention, the content ratio of the urethane polymer and the oily component is not particularly limited, but the content ratio of the urethane polymer and the oily component is, for example, 0.1:99.9 to 95:5 in terms of mass ratio (the total in the mass ratio is 100).

Fields in which the oil composition of the present invention can be used are not particularly limited, but this oil composition can be used in, for example, coating materials, adhesives, fuels, lubricants, foods and cosmetic products. In such cases, well-known materials may be contained in coating materials, adhesives, fuels, lubricants, foods and cosmetic products according to intended uses and purposes thereof.

In the oil composition of the present invention, the total amount of the urethane polymer and the oily component is not particularly limited, but is generally 80 mass% or more relative to the total amount of the oil composition, preferably 90 mass% or more, more preferably 95 mass% or more, and further preferably 99 mass% or more, and it is most preferable for the oil composition of the present invention to consist of the urethane polymer and the oily component.

Moreover, components other than the urethane polymer and the oily component contained in the oil composition of the present invention are selected as appropriate according to the intended use and purpose of the oil composition.

The cosmetic composition of the present invention contains the oil composition of the present invention. According to the present invention, it is possible to obtain a cosmetic composition whose viscosity and feel before and after use are modified or improved. In the cosmetic composition of the present invention, the content of the oil composition is not particularly limited, but it is preferable to incorporate the oil composition of the present invention at a quantity of, for example, 0.01 to 90 parts by mass relative to 100 parts by mass of the cosmetic composition.

Other commonly used additives can be used in the cosmetic composition of the present invention in order to improve or modify a variety of characteristics (for example, solubility, dispersibility, stability, feeling of use, applicability, permeability, moisturizing properties, safety, aesthetic properties, optical characteristics, aromatic properties and whitening properties) of the cosmetic composition during storage, during use or after use according to the intended use of the cosmetic composition. Examples of these other additives include powder components, liquid oils/fats, solid oils/fats, waxes, higher fatty acids, anionic surfactants, cationic surfactants, amphoteric surfactants, non-ionic surfactants, humectants, polymer compounds, metal ion sequestering agents, lower alcohols, polyhydric alcohols, sugars, amino acids and derivatives thereof, organic amines, pH adjusting agents, antioxidants, preservatives, blood circulation promoters, antiphlogistic agents, activators, whitening agents, antiseborrheic agents, anti-inflammatory agents and a variety of extracts and plant seaweed extracts, and one or more types of these can be blended according to need.

In addition, specific applications of the cosmetic composition of the present invention are not particularly limited, and include, for example, toiletry products such as shampoos and conditioners, and examples of these applications include skin lotions, cosmetic liquids, milky lotions, creams, face washing foams, cleansing milks, cleansing lotions, hair tonics, hair liquids, setting lotions, hair bleaches, color rinses, permanent wave liquids, lipsticks, packs, foundations, eaux de cologne, shampoos, conditioners, treatments, sunscreens, deodorants, perfumes, cleansing oils and cosmetic oils.

### Examples

The present invention will now be explained in greater detail with reference to of examples and comparative examples, but the present invention is in no way limited to these examples. Moreover, in the examples etc. given below, % means mass% unless explicitly indicated otherwise.

### <Production of alkylene oxide-added alcohol 1>

1000 g of 1-dodecanol and 20.1 g of potassium hydroxide as a catalyst were placed in a 10 liter autoclave and dissolved, after which 6191.6 g of butylene oxide (corresponding to 16 times the mass of introduced 1-dodecanol) was slowly added in an environment having a temperature of 100 to 140°C and a pressure of 1.0 to 6.0 kg/cm², and waiting until an addition reaction had progressed and the pressure in the reaction vessel became constant, an addition reaction of a total of 16 moles of butylene oxide with 1 mole of 1-dodecanol was completed. Finally, potassium hydroxide as a catalyst was treated with an adsorbent, thereby obtaining alkylene oxide-added alcohol 1 (AO-added alcohol 1).

### <Production of alkylene oxide-added alcohols 2 to 8>

Alkylene oxide-added alcohols 2 to 8 were obtained by carrying out an addition reaction using a similar method to that used for producing alkylene oxide-added alcohol 1, except that the raw materials used and/or AO addition number were altered as shown in Table 1. Moreover, the AO addition number in Table 1 is the number of moles of alkylene oxide added to 1 mole of an alcohol compound. In addition, alkylene oxide-added alcohol 7 is a compound obtained by adding 9 moles of propylene oxide to methanol and then adding 7 moles of butylene oxide, and alkylene oxide-added alcohol 8 is a compound consisting of a propylene glycol polymer (polypropylene glycol) obtained by polymerizing propylene oxide with a water molecule.

| | Alcohol compound | Alkylene oxide | AO addition number |
|---|---|---|---|
| AO-added alcohol 1 | 1-dodecanol | Butylene oxide | 16 |
| AO-added alcohol 2 | 1-dodecanol | Butylene oxide | 30 |
| AO-added alcohol 3 | 1-octadecanol | Butylene oxide | 16 |
| AO-added alcohol 4 | C6 diol | Butylene oxide | 16 |
| AO-added alcohol 5 | C36 diol | Butylene oxide | 16 |
| AO-added alcohol 6 | 1-dodecanol | Propylene oxide | 16 |
| AO-added alcohol 7 | Methanol | Propylene oxide | 9 |
| | | Butylene oxide | 7 |
| AO-added alcohol 8 | | Propylene oxide | 12 |

### <Production of urethane polymer 1>

Urethane polymer 1 was produced by reacting alkylene oxide-added alcohol 1 and isophorone diisocyanate under conditions such that the equivalence ratio of hydroxyl groups in alkylene oxide-added alcohol 1 and isocyanate groups in isophorone diisocyanate was 0.5. Specifically, urethane prepolymer 1 was obtained by charging 959.9 g of alkylene oxide-added alcohol 1 and 40.1 g of isophorone diisocyanate in a four-mouthed flask having a capacity of 2000 mL and equipped with a temperature gauge, a nitrogen inlet tube and a stirrer, mixing the reactants until homogeneous while purging the system with nitrogen, and then reacting for three hours at 100 to 120°C. Table 3 gives information on the structure of urethane polymer 1 in relation to general formula (1).

### (Diisocyanate compounds used)

IPDI: Isophorone diisocyanate
H12MDI: Dicyclohexylmethane-4,4'-diisocyanate
HDI: Hexamethylene diisocyanate

### <Production of urethane polymers 2 to 14>

Urethane polymers 2 to 14 were obtained by reacting an alkylene oxide-added alcohol compound with a diisocyanate compound using a method similar to that used in the production method of urethane polymer 1, except that raw materials and blending proportions were as shown in Table 2. Moreover, equivalence ratios in Table 2 indicate equivalence ratios of hydroxyl groups in the AO-added alcohols used and isocyanate groups in the diisocyanates used. Table 3 and Table 4 give information on the structures of urethane polymers 2 to 14 in relation to general formula (1). Moreover, urethane polymers 7 and 8 and urethane polymers 9 and 10, which differ in terms of equivalence ratio of AO-added alcohol and diisocyanate compound used, were equivalent in terms of the distribution range for the value of n in general formula (1), but the abundance ratio of compounds having these values of n differed slightly.

| | AO-added alcohol | Diisocyanate compound | Equivalence ratio |
|---|---|---|---|
| Urethane polymer 1 | AO-added alcohol 1 | IPDI | 1:0.5 |
| Urethane polymer 2 | AO-added alcohol 1 | H12MDI | 1:0.5 |
| Urethane polymer 3 | AO-added alcohol 2 | IPDI | 1:0.5 |
| Urethane polymer 4 | AO-added alcohol 2 | H12MDI | 1:0.5 |
| Urethane polymer 5 | AO-added alcohol 2 | HDI | 1:0.5 |
| Urethane polymer 6 | AO-added alcohol 3 | IPDI | 1:0.5 |
| Urethane polymer 7 | AO-added alcohol 4 | IPDI | 1:0.25 |
| Urethane polymer 8 | AO-added alcohol 4 | IPDI | 1:0.5 |
| Urethane polymer 9 | AO-added alcohol 5 | IPDI | 1:0.25 |
| Urethane polymer 10 | AO-added alcohol 5 | IPDI | 1:0.5 |
| Urethane polymer 11 | AO-added alcohol 6 | H12MDI | 1:0.5 |
| Urethane polymer 12 | AO-added alcohol 7 | H12MDI | 1:0.5 |
| Urethane polymer 13 | AO-added alcohol 8 | H12MDI | 1:0.5 |
| Urethane polymer 14 | AO-added alcohol 8 | IPDI | 1:0.5 |

| | R¹, R² | Z¹ | Z² | R³ | X¹ | X² | n |
|---|---|---|---|---|---|---|---|
| Urethane polymer 1 | Dodecylene group | Group in which a₁:b₁:c₁=1:0:0 and p is 16 in general formula (2) | Group in which a₂:b₂:c₂=1:0:0 and q is 16 in general formula (3) | IPDI residue | Hydrogen atom | Hydrogen atom | 1 |
| Urethane polymer 2 | Dodecylene group | Group in which a₁:b₁:c₁=1:0:0 and p is 16 in general formula (2) | Group in which a₂:b₂:c₂=1:0:0 and q is 16 in general formula (3) | H12MDI residue | Hydrogen atom | Hydrogen atom | 1 |
| Urethane polymer 3 | Dodecylene group | Group in which a₁:b₁:c₁=1:0:0 and p is 30 in general formula (2) | Group in which a₂:b₂:c₂=1:0:0 and q is 30 in general formula (3) | IPDI residue | Hydrogen atom | Hydrogen atom | 1 |
| Urethane polymer 4 | Dodecylene group | Group in which a₁:b₁:c₁=1:0:0 and p is 30 in general formula (2) | Group in which a₂:b₂:c₂=1:0:0 and q is 30 in general formula (3) | H12MDI residue | Hydrogen atom | Hydrogen atom | 1 |
| Urethane polymer 5 | Dodecylene group | Group in which a₁:b₁:c₁=1:0:0 and p is 30 in general formula (2) | Group in which a₂:b₂:c₂=1:0:0 and q is 30 in general formula (3) | HDI residue | Hydrogen atom | Hydrogen atom | 1 |
| Urethane polymer 6 | Octadecylene group | Group in which a₁:b₁:c₁=1:0:0 and p is 16 in general formula (2) | Group in which a₂:b₂:c₂=1:0:0 and q is 16 in general formula (3) | IPDI residue | Hydrogen atom | Hydrogen atom | 1 |
| Urethane polymer 7 | Hexylene group | Group in which a₁:b₁:c₁=1:0:0 and p is 8 in general formula (2) | Group in which a₂:b₂:c₂=1:0:0 and q is 8 in general formula (3) | IPDI residue | Group in which a₃:b₃:c₃=1:0:0 and r is 8 in general formula (4) | Group in which a₄:b₄:c₄=1:0:0 and s is 8 in general formula (5) | 1-10 |
| Urethane polymer 8 | Hexylene group | Group in which a₁:b₁:c₁=1:0:0 and p is 8 in general formula (2) | Group in which a₂:b₂:c₂=1:0:0 and q is 8 in general formula (3) | IPDI residue | Group in which a₃:b₃:c₃=1:0: 0 and r is 8 in general formula (4) | Group in which a₄:b₄:c₄=1:0:0 and s is 8 in general formula (5) | 1-10 |
| Urethane polymer 9 | Alkylene group with 36 carbon atoms | Group in which a₁:b₁:c₁=1:0:0 and p is 8 in general formula (2) | Group in which a₂:b₂:c₂=1:0:0 and q is 8 in general formula (3) | IPDI residue | Group in which a₃:b₃:c₃=1:0: 0 and r is 8 in general formula (4) | Group in which a₄:b₄:c₄=1:0:0 and s is 8 in general formula (5) | 1-10 |
| Urethane polymer 10 | Alkylene group with 36 carbon atoms | Group in which a₁:b₁:c₁=1:0:0 and p is 8 in general formula (2) | Group in which a₂:b₂:c₂=1:0:0 and q is 8 in general formula (3) | IPDI residue | Group in which a₃:b₃:c₃=1:0: 0 and r is 8 in general formula (4) | Group in which a₄:b₄:c₄=1:0:0 and s is 8 in general formula (5) | 1-10 |
| Urethane polymer 11 | Dodecylene group | Group in which a₁:b₁:c₁=0:1:0 and p is 16 in general formula (2) | Group in which a₂:b₂:c₂=0:1:0 and q is 16 in general formula (3) | H12MDI residue | Hydrogen atom | Hydrogen atom | 1 |
| Urethane polymer 12 | Methylene group | Group in which a₁:b₁:c₁=0.44:0.56:0 and p is 16 in general formula (2) | Group in which a₂:b₂:c₂=0.44:0.36:0 and q is 16 in general formula (3) | H12MDI residue | Hydrogen atom | Hydrogen atom | 1 |
| Urethane polymer 13 | - | Group in which a₁:b₁:c₁=0:1:0 and p is 12 in general formula (2) | Group in which a₂:b₂:c₂=0:1:0 and q is 12 in general formula (3) | H12MDI residue | Hydrogen atom | Hydrogen atom | 1 |
| Urethane polymer 14 | | Group in which a₁:b₁:c₁=0:1:0 and p is 12 in general formula (2) | Group in which a₂:b₂:c₂=0:1:0 and q is 12 in general formula (3) | IPDI residue | Hydrogen atom | Hydrogen atom | 1 |

### <Solubility evaluation in oily components 1>

Obtained urethane polymers 1 to 14 were evaluated in terms of solubility in oily components. Specifically, 5 g of a urethane polymer and 20 g of an oily component (that is, a mass ratio of 20:80), as shown in Table 5, were added to a 100 mL glass beaker so as to prepare an oil composition, and the oil composition was homogenized by being shaken at room temperature and then allowed to rest for 24 hours in a constant temperature bath at 25°C. After resting, the appearance of each oil composition was visually observed, and solubility was evaluated on the basis of the following evaluation criteria. The evaluation results are shown in Table 5.

### (Oily components used)

Oily component 1: Hydrogenated polyisobutene (hydrocarbon oil)
Oily component 2: Diphenylsiloxy phenyl trimethicone (chain-like silicone oil)
Oily component 3: Cyclopentasiloxane (cyclic silicone oil)
Oily component 4: Isononyl isononanoate (low polarity ester oil)
Oily component 5: Ethylhexyl hydroxystearate (high polarity ester oil)
Oily component 6: Triethylhexanoin (triglyceride)

### (Solubility evaluation criteria)

○: Completely dissolved (transparent)
×: White turbidness observed

| | Solubility evaluation results (mass ratio 20:80) | | | | | |
|---|---|---|---|---|---|---|
| | Oily component 1 | Oily component 2 | Oily component 3 | Oily component 4 | Oily component 5 | Oily component 6 |
| Urethane polymer 1 | ○ | ○ | ○ | ○ | ○ | ○ |
| Urethane polymer 2 | ○ | ○ | ○ | ○ | ○ | ○ |
| Urethane polymer 3 | ○ | ○ | ○ | ○ | ○ | ○ |
| Urethane polymer 4 | ○ | ○ | ○ | ○ | ○ | ○ |
| Urethane polymer 5 | ○ | ○ | ○ | ○ | ○ | ○ |
| Urethane polymer 6 | ○ | ○ | ○ | ○ | ○ | ○ |
| Urethane polymer 7 | ○ | ○ | ○ | ○ | ○ | ○ |
| Urethane polymer 8 | ○ | ○ | ○ | ○ | ○ | ○ |
| Urethane polymer 9 | ○ | ○ | ○ | ○ | ○ | ○ |
| Urethane polymer 10 | ○ | ○ | ○ | ○ | ○ | ○ |
| Urethane polymer 11 | ○ | ○ | × | ○ | ○ | ○ |
| Urethane polymer 12 | ○ | ○ | × | ○ | ○ | ○ |
| Urethane polymer 13 | × | ○ | × | ○ | ○ | ○ |
| Urethane polymer 14 | × | ○ | × | ○ | ○ | ○ |

### <Solubility evaluation in oily components 2>

Oil compositions were prepared and solubility in oily components was evaluated using a method similar to that used in the solubility evaluation in oily components 1, except that blending conditions of the urethane polymer and the oily component were changed as shown in Table 6. The evaluation results are shown in Table 6.

| | Solubility evaluation results | | | | | |
|---|---|---|---|---|---|---|
| | Oily component 1 | | | Oily component 3 | | |
| | Mass ratio 1:99 | Mass ratio 10:90 | Mass ratio 90:10 | Mass ratio 1:99 | Mass ratio 10:90 | Mass ratio 90:10 |
| Urethane polymer 1 | ○ | ○ | ○ | ○ | ○ | ○ |
| Urethane polymer 3 | ○ | ○ | ○ | ○ | ○ | ○ |
| Urethane polymer 6 | ○ | ○ | ○ | ○ | ○ | ○ |
| Urethane polymer 13 | × | × | × | × | × | × |
| Urethane polymer 14 | × | × | × | × | × | × |

### <Evaluation of characteristics of oil compositions>

An oil composition consisting of 5 g of the urethane polymer of the present invention and 20 g of an oily component (that is, a mass ratio of 20:80) and, as comparative examples, oil compositions consisting only of oily components 1 to 6 were prepared as shown in Table 7, and feel and glossiness were evaluated as characteristics of the oil compositions. Specifically, feel when a prepared oil composition was scooped up using a finger and skin luster (glossiness) after an oil composition was applied to skin were evaluated according to the following evaluation criteria. The evaluation results are shown in Table 7.

### (Feel evaluation criteria)

○: Smooth feel
×: No smooth feel
(Glossiness evaluation criteria)
○: Excellent skin luster
×: No skin luster whatsoever

| | | Evaluation of characteristics of oil compositions (mass ratio 20:80) | | | | | |
|---|---|---|---|---|---|---|---|
| | | Oily component 1 | Oily component 2 | Oily component 3 | Oily component 4 | Oily component 5 | Oily component 6 |
| Urethane polymer 1 | Feel | ○ | ○ | ○ | ○ | ○ | ○ |
| | Glossiness | ○ | ○ | ○ | ○ | ○ | ○ |
| Urethane polymer 3 | Feel | ○ | ○ | ○ | ○ | ○ | ○ |
| | Glossiness | ○ | ○ | ○ | ○ | ○ | ○ |
| Urethane polymer 6 | Feel | ○ | ○ | ○ | ○ | ○ | ○ |
| | Glossiness | ○ | ○ | ○ | ○ | ○ | ○ |
| Oily component only | Feel | × | × | × | × | × | × |
| | Glossiness | × | × | × | × | × | × |

As can be understood from the results above, it has been confirmed that the urethane polymer of the present invention is soluble in a variety of oily components, such as hydrocarbon oils, silicone oils and ester oils, and can adjust characteristics such as feel before and after use. According to the present invention, it is possible to obtain: a urethane polymer which is soluble in a variety of oily components, such as hydrocarbon oils, silicone oils and ester oils, and can therefore modify or improve characteristics, such as viscosity and feel before and after use, of a variety of products that contain oily components, such as coating materials, adhesives, fuels, lubricants, foods and cosmetic products; and an oil composition whose viscosity and feel before and after use are modified or improved.

## Claims

1. A urethane polymer having a structure represented by general formula (1) below: 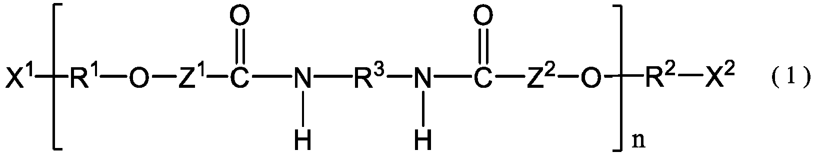 wherein R¹ and R² each independently denote an alkylene group having 5 to 40 carbon atoms, Z¹ and Z² each denote an oxyalkylene group represented by general formula (2) or (3) below, R³ denotes a diisocyanate residue, X¹ denotes a hydrogen atom or a group represented by general formula (4) below, X² denotes a hydrogen atom or a group represented by general formula (5) below, and n denotes a number from 1 to 20, provided that n is 1 if X¹ and X² are each a hydrogen atom; 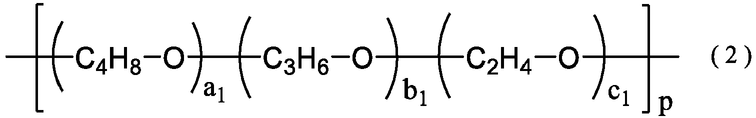 wherein a₁, b₁ and c₁ denote a ratio a₁:b₁:c₁ = 1-0.6:0-0.4:0-0.4 (a sum of a₁, b₁ and c₁ is 1), and p denotes a number from 4 to 60 and wherein arrangement of structural units may be block-like or random; 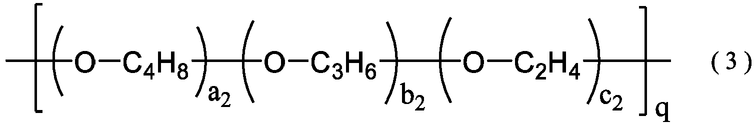 wherein a₂, b₂ and c₂ denote a ratio a₂:b₂:c₂ = 1-0.6:0-0.4:0-0.4 (a sum of a₂, b₂ and c₂ is 1), and q denotes a number from 4 to 60 and wherein arrangement of structural units may be block-like or random; 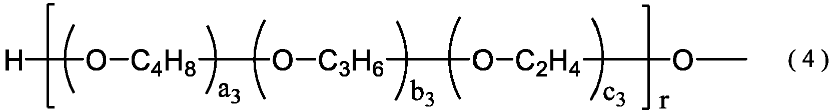 wherein a₃, b₃ and c₃ denote a ratio a₃:b₃:c₃ = 1-0.6:0-0.4:0-0.4 (a sum of a₃, b₃ and c₃ is 1), and r denotes a number from 4 to 60 and wherein arrangement of structural units may be block-like or random; 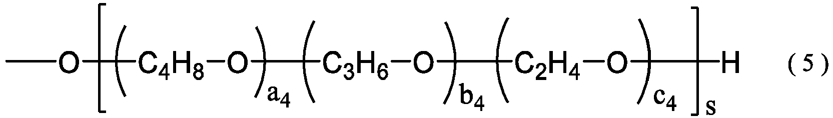 wherein a₄, b₄ and c₄ denote a ratio a₄:b₄:c₄ = 1-0.6:0-0.4:0-0.4 (a sum of a₄, b₄ and c₄ is 1), and s denotes a number from 4 to 60 and wherein arrangement of structural units may be block-like or random.

2. The urethane polymer according to claim 1, wherein Z¹ and Z² are each an oxyalkylene group consisting only of an oxybutylene unit.

3. The urethane polymer according to claim 1 or claim 2, wherein Z¹ is a group in which p is a number from 6 to 40 in general formula (2) and Z² is a group in which q is a number from 6 to 40 in general formula (3).

4. The urethane polymer according to any one of claims 1 to 3, wherein R³ is an aliphatic hydrocarbon group having 4 to 16 carbon atoms or an alicyclic hydrocarbon group having 4 to 16 carbon atoms.

5. The urethane polymer according to any one of claims 1 to 4, wherein X¹ and X² are each a hydrogen atom and n is 1.

6. An oil composition containing the urethane polymer according to any one of claims 1 to 5 and an oily component that includes at least one type selected from the group consisting of a hydrocarbon oil, a silicone oil and an ester oil.

7. The oil composition according to claim 6, wherein a content ratio of the urethane polymer and the oily component is 0.1:99.9 to 95:5 in terms of mass ratio (a total of the mass ratio is 100.).

8. A cosmetic composition, containing the oil composition according to claim 6 or claim 7.

9. A method for improving luster of a cosmetic composition that contains an oily component, the method comprising combining the urethane polymer according to any one of claims 1 to 5 with the oily component, which includes at least one type selected from the group consisting of a hydrocarbon oil, a silicone oil and an ester oil.

10. Use of the urethane polymer according to any one of claims 1 to 5 to improve luster of a cosmetic composition that contains an oily component that includes at least one type selected from the group consisting of a hydrocarbon oil, a silicone oil and an ester oil, wherein the urethane polymer is dissolved in the oily component.
